Europäisches Patentamt

European Patent Office

Office européen des brevets

(11) Veröffentlichungsnummer : **0 149 628 B1**

(12) **EUROPÄISCHE PATENTSCHRIFT**

(45) Veröffentlichungstag der Patentschrift :
17.08.88

(21) Anmeldenummer : 84902505.1

(22) Anmeldetag : 12.06.84

(86) Internationale Anmeldenummer :
PCT/DE 84/00132

(87) Internationale Veröffentlichungsnummer :
WO/8500420 (31.01.85 Gazette 85/03)

(51) Int. Cl.⁴ : **F 24 H  1/00, A 61 M  1/36, A 23 L  3/00**

(54) VERFAHREN UND VORRICHTUNG ZUR TEMPERATURREGELUNG VON FLÜSSIGKEITEN.

(30) Priorität : 13.07.83 DE 3325195

(43) Veröffentlichungstag der Anmeldung :
31.07.85 Patentblatt 85/31

(45) Bekanntmachung des Hinweises auf die Patenterteilung : 17.08.88 Patentblatt 88/33

(84) Benannte Vertragsstaaten :
CH DE FR GB LI SE

(56) Entgegenhaltungen :
DE-A- 2 241 681
DE-B- 1 037 830
GB-A- 1 179 827
GB-A- 1 269 606

(73) Patentinhaber : HAGELAUER, Ulrich
Magirusstrasse 23
D-7000 Stuttgart-Feuerbach (DE)

FAUST, Uwe
Relenbergstrasse 57
D-7000 Stuttgart 1 (DE)

(72) Erfinder : HAGELAUER, Ulrich
Magirusstrasse 23
D-7000 Stuttgart-Feuerbach (DE)
Erfinder : FAUST, Uwe
Relenbergstrasse 57
D-7000 Stuttgart 1 (DE)

(74) Vertreter : Schuster, Gregor, Dipl.-Ing.
Patentanwälte Schuster & Thul Wiederholdstrasse 10
D-7000 Stuttgart 1 (DE)

**Beschreibung**

Die Erfindung geht aus von einem Verfahren zur Temperaturregelung einer Flüssigkeit mit elektrischen Mitteln und Steuerung der elektrischen Mittel unter Auswertung des Temperaturvergleichs der gemessenen Ist-Temperatur mit einer Soll-Temperatur und unter Änderung der an die Flüssigkeit abgegebenen Wärmeleistung bzw. von einer Vorrichtung zur Durchführung dieses Verfahrens mit einem topf- oder rohrförmigen Flüssigkeitsbehälter bestimmten Volumens, mit einem elektrischen Wärmeerzeuger für die im Behälter vorhandene Flüssigkeit, mit einem Temperaturgeber zur Messung der Ist-Temperatur und mit einem mit dem Temperaturgeber und dem Wärmeerzeuger elektrisch verbundenen elektronischen Steuergerät zur Änderung der Wärmeleistung nach Vergleich und Auswertung der gemessenen Ist-Temperatur mit einer vorgegebenen Soll-Temperatur.

Bei den in vielfältiger Weise bekannten Verfahren oder Vorrichtungen dieser Art wird die Flüssigkeit erwärmt, indem entweder ein Heizelement unmittelbar in die Flüssigkeit ragt oder indem ein Heizelement eine Behälterwand aufheizt und mittelbar dadurch die in dem Behälter enthaltende Flüssigkeit. Aufgrund dieses Konvektionsheizungsprinzips entstehen partielle « Überhitzungen » zumindest so lange, bis sich die Wärme gleichmäßig verteilt hat. Ganz abgesehen davon, daß eine derartige Aufheizung einen relativ ungünstigen Wirkungsgrad aufweist und zudem verhältnismäßig lang dauert, kann die partielle Überhitzung zur Zerstörung, insbesondere empfindlicher organischer Reagenzien an den Wärmeaustauschflächen führen. Hinzu kommt der Nachteil, daß derartige Wärmeerzeuger einen Nachheizeffekt haben, der auf der Wärmespeicherwirkung des Wärmeerzeugermaterials beruht. Eine exakte Regelung, bei der die Soll-Temperatur nicht überschritten wird, ist dadurch nahezu unmöglich.

Andere Temperaturregelungsverfahren für Flüssigkeiten unter der Verwendung von Mikrowellen als elektrische Mittel oder Infrarotstrahlung haben den Nachteil, daß Mikrowellen die Temperatur des messenden Gebers beeinflussen und daß die Infrarotstrahlung aufgrund der hohen spezifischen Absorption mit zunehmender Eindringtiefe in die Flüssigkeit an Intensität schnell abnimmt, so daß auch hier örtliche Überhitzungen eintreten.

Bei allen diesen bekannten Temperaturregelungen von Flüssigkeiten wird durch die ungleichmäßige Erwärmung in manchen Abschnitten des zu erwärmenden Flüssigkeitsvolumens die Soll-Temperatur überschritten. Bei einer angestreben kurzen Temperaturregelungszeit sind diese partiellen Temperaturüberschreitungen der Soll-Temperatur verhältnismäßig hoch, so daß empfindliche Flüssigkeiten gefährdet sind. Bei nur geringen Überschreitungen der Soll-Temperatur ist die Temperaturregelzeit entsprechend lang, länger als es für die meisten praktischen Einsätze vertretbar ist.

Der Erfindung liegt demgegenüber die Aufgabe zugrunde, ein Verfahren bzw. eine Vorrichtung zu entwickeln, durch die eine Temperaturregelung besonders von empfindlichen Flüssigkeiten erzielt wird, mit einer sehr genauen Einhaltung der Soll-Temperatur ohne auch nur partielle Überschreitung derselben und wobei diese Soll-Temperatur sehr schnell erreicht wird.

Diese Aufgabe wird erfindungsgemäß durch das Verfahren gelöst, daß akustische Ultraschallwellen durch die elektrischen Mittel erzeugt werden und die Änderung der Ist-Temperatur entsprechend der vorgegebenen Solltemperatur durch Ändern der Schwingungsamplitude oder durch Ein- und Ausschalten des Ultraschalls erfolgt bzw. durch eine Vorrichtung, bei der zu dieser Temperaturregelung der Behälter mit einem einen elektrischen Generator zur Erzeugung der Resonanzfrequenz aufweisenden elektroakustischen Wandler verbunden ist, wobei der Temperaturgeber aufgrund der Ultraschallhomogenisierung der Flüssigkeit an einer beliebigen Stelle des Volumens angeordnet ist und wobei durch das elektronische Steuergerät die Leistung des Generators bis zum Erreichen der Soll-Temperatur änderbar oder abschaltbar ist.

Die Erfindung baut auf die bisher unbekannte Eigenschaft von Ultraschall auf, daß die Temperaturänderungen eines bestimmten Flüssigkeitsvolumens bei entsprechend geringer Ultraschallintensität kleiner sein können als sie von den bekannten Temperaturmeßmethoden erfaßbar sind, so daß die einzuregelnde Soll-Temperatur außerordentlich genau eingehalten werden kann. Diese Feinheit in der Temperaturregelung ist auch nur deshalb möglich, weil der Ultraschall die bekannte homogenisierende Wirkung aufweist. So ist der Vorteil der Erfindung besonders in der Medizintechnik gegeben, bei der empfindliche organische Reagenzien eine schnelle Aufheizung ohne partielle Überhitzung erfahren können, die zur Zerstörung der Flüssigkeit, die beispielsweise biologisches Material wie Proteine und lebende Zellen enthält, führen würde. Es ist zwar bekannt (« Die Lebensmittel-Industrie » 1967, S. 219 bis 223), daß Ultraschall eine homogenisierende Wirkung auf Flüssigkeiten hat oder daß Ultraschall eine Erwärmung der Flüssigkeit bewirkt. Es ist ebenfalls so bekannt, Ultraschall bei der Herstellung von Speiseeis, Margarine, Suppen usw. unter Nutzung des Vorteils der Homogenisierung einzusetzen, wobei allerdings die Erwärmung als notwendiges Übel in Kauf genommen wird.

Ein weiterer Vorteil der Erfindung besteht darin, daß die Ist-Temperaturmessung an irgendeiner Stelle der Flüssigkeit erfolgen kann, da aufgrund der homogenisierenden Wirkung die Flüssigkeit überall die gleiche Temperatur aufweist. So ist das Verfahren vorteilhafterweise bei ruhenden sowie strömenden Flüssigkeiten einsetzbar, auch wenn die mögliche Wärmeabfuhr aus der Flüssigkeit unterschiedlich ist. Bei strömenden Flüssigkeiten kann die Zuströmtemperatur der Flüssigkeit, bei ruhenden Flüssigkeiten die am Behälter auf den verschiedenen Seiten anliegende Umgebungstemperatur unterschiedlich sein. So ist auch denkbar, daß die Umgebungstemperatur höher ist als die Temperatur der beschallten Flüssigkeit oder auch deren angestrebte Soll-Temperatur, so lange die Einwirkung der Umge-bungstemperatur auf die Flüssigkeit vernachläs-sigbar ist, was meist der Fall ist. Die Ist-Temperatur hingegen muß grundsätzlich niedriger sein als die Soll-Temperatur, da die Regelung nur über eine Erwärmung der Flüssigkeit möglich ist.

Ein weiterer Vorteil der Erfindung besteht darin, daß die Steuerung des Ultraschalls trägheitslos erfolgt, so daß keine aufwendigen elektronischen Steuergeräte erforderlich sind und meist eine einfache Proportionalregelung genügt.

Weitere Vorteile und Ausgestaltungen der Erfindung sind der nachfolgenden Beschreibung, der Zeichnung und den Ansprüchen entnehmbar.

Vier Ausführungsbeispiele des Gegenstandes der Erfindung sind in der Zeichnung näher erläutert und im folgenden näher beschrieben...

Fig. 1 und 2 das erste Ausführungsbeispiel einer Durchflußtemperierung

Fig. 3 das zweite Ausführungsbeispiel als Temperierung in Laborgefäßen Fig. 4 das dritte Ausführungsbeispiel als Temperierung abgeschlossener Flüssigkeitsvolumina und

Fig. 5 das vierte Ausführungsbeispiel als Temperierung durch Erregung der Wandung eines topfförmigen Behälters.

Bei allen nachfolgend beschriebenen Beispielen handelt es sich um Geräte zur Ultraschallerzeugung und Temperierung von Flüssigkeiten, denen jeweils ein elektronisches Steuergerät zugeordnet ist, das hier nicht näher beschrieben ist. Diese elektronischen Steuergeräte arbeiten in bekannter Weise, indem das Signal einer Ist-Tem-peraturmessung eingegeben wird und entspre-chend einem Programm nach Vergleich mit einem Soll-Temperaturwert elektroakustische Erreger steuert, so daß endgültig eine Regelung der Temperatur bzw. eine Temperierung erzielbar ist.

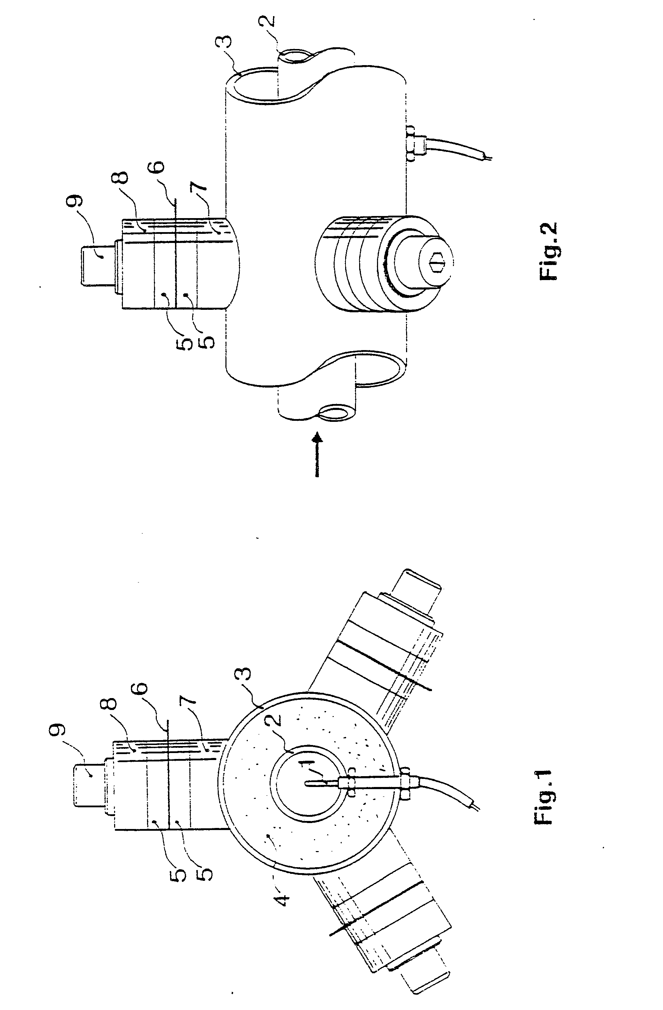

Mit dem in den Fig. 1 und 2 dargestellten Gerät wird über einen Temperaturgeber 1 die Ist-Temperatur der durch ein Rohr 2 strömenden und zu temperierenden Flüssigkeit gemessen. Koaxial ist um dieses Rohr 2 ein hülsenförmiges Abstrahlglied 3 angeordnet, wobei zwischen Rohr 2 und Hülse 3 ein Koppelmedium 4 vorgesehen ist.

Das Koppelmedium dient neben der Schallübertragung auch der thermischen Isolation, so daß bei hohen Temperaturen vom Schwingungserzeuger nach Abstellen keine Temperatur auf das zu temperierende Medium übergeht. Natürlich dürfen als Koppelmedium keine Gase dienen, da sonst sofort eine Totalreflektion des Ultraschalls gegeben wäre.

Auf der Abstrahlgliedhülse sind auf den Umfang gleichmässig verteilt drei elektroakustisch arbeitende Ultraschallverbundschwinger angeordnet

mit jeweils zwei elektroakustischen Wandlern 5, zwischen denen eine Kontaktscheibe 6 angeordnet ist, einem Adapter 7 zur Hülse 3 hin, einem auf der anderen Seite des Verbundschwingers angeordnetem Abschlußglied 8 und einer die Teile zusammenhaltenden Verschraubung 9. Die elektroakustischen Wandler 5 können aus einer beliebigen Anzahl von piezokeramischen oder magnetostriktiven oder ähnlichen Teilen bestehen. Die Kontaktscheibe 6, die vorzugsweise aus Kupferberyllium besteht, dient als Anschlußteil der elektroakustischen Wandler 5 an einen elektrischen Generator, d. h. zur Erregung der Ultraschall-Erzeuger auf deren Resonanzfrequenz.

Wie aus Fig. 2 und der dort über den Pfeil dargestellten Strömungsrichtung der Flüssigkeit entnehmbar, ist der Temperaturgeber 1 stromab der Ultraschallschwinger 5-9 angeordnet. Somit ist die im Rohr 2 gemessene Ist-Temperatur bereits durch die Ultraschallsteuerung bestimmbar. Stärke und Dauer des Ultraschalls kann zur Temperierung somit durch das elektronische Steuergerät bestimmt werden. Es ist jedoch auch denkbar, daß mehrere derartige Einheiten hintereinander auf dem Rohr 2 angeordnet sind. Die Resonanzfrequenz läßt sich durch Wahl geeigneter Werkstoffe, Abmessungen und Anzahl derartiger Verbundschwinger erzielen, wobei sich eine homogene Energieeinkoppelung im Transportrohr 2 ergibt.

Durch das Zwischenschalten eines Koppelmediums 4 wird erreicht, daß die Resonanzerscheinungen im Transportrohr 2 bzw. im Koppelmedium 4 mit ausgenutzt werden, um dadurch einen möglichst hohen Energieanteil einzukoppeln.

Durch die sternartige Anordnung der Ultraschallverbundschwinger (siehe Fig. 1) wird zudem der Effekt der Ultraschallbündelung durch die den Verbundschwingern gegenüberliegenden Wände ausgenutzt. Auf diese Weise durchläuft die Ultraschallwelle mehrfach das Transportrohr.

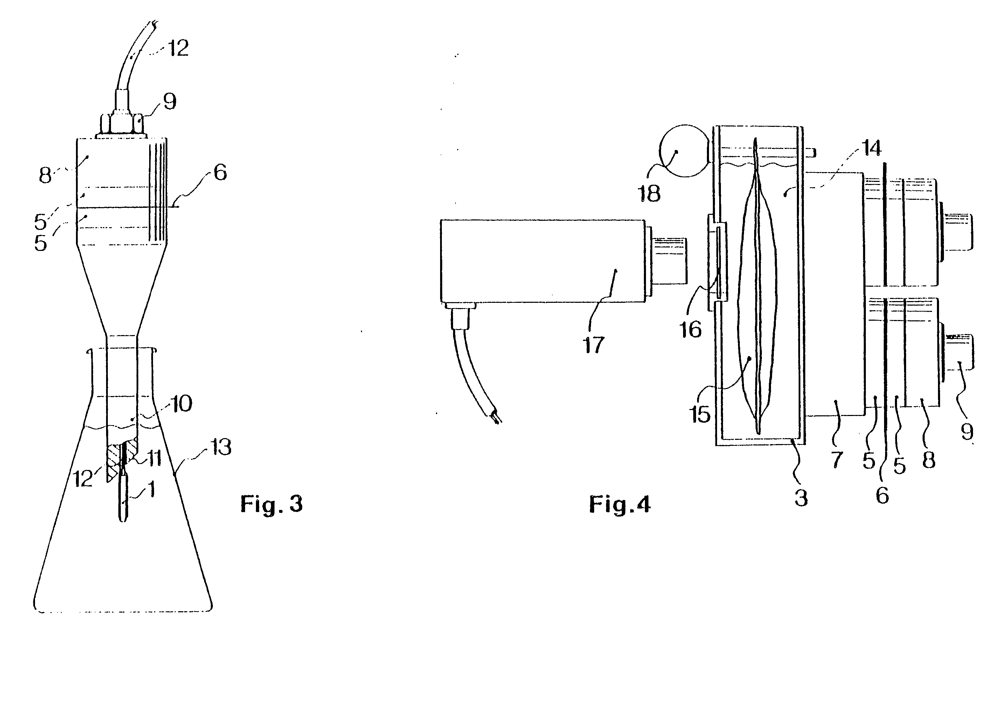

In Fig. 3 ist als weiteres Beispiel für die Anwendung in Laboratorien ein « handlicher Ultraschallerzeuger » dargestellt. Der Aufbau des Gerätes entspricht im wesentlichen den Teilen 5, 6, 8 und 9 des oben beschriebenen Beispiels. Lediglich statt des Adapters 7 ist hier ein Abstrahlhorn 10 vorgesehen, das auf der dem Wandler 5 abgewandten und verjüngten Seite eine Abstrahlfläche 11 aufweist, die schräg zur Geräteachse verläuft. Der ganze Schwinger ist dann zentral axial durchbohrt, um ein zum Temperaturgeber 1 führendes Kabel 12 aufzunehmen. Das Abstrahlhorn 10 mit Strahlfläche 11 wird in die zu behandelnde Flüssigkeit getaucht, die sich beispielsweise in einem Laborgefäß 13 befindet. Durch Gestaltung der turbulenzerzeugenden Fläche 11 wird in Bezug auf die Form des Laborgefäßes 13 eine optimale und möglichst Verlustarme Ultraschallbehandlung der Flüssigkeit erzielt. Die über den Temperaturgeber 1 im Gefäß gemessene Flüssigkeit-stemperatur wird dem nichtgezeigten elektroni-schen Steuergerät eingegeben und dort mit dem Sollwert verglichen. Bei einem Laborgerät kann beispielsweise ein solcher Sollwert willkürlich jeweils eingestellt werden. Gemäß dem eingegebenen Programm, beispielsweise einer speziellen Regelgröße, ergibt die Temperaturdifferenz aus Ist- und Sollwert ein Steuersignal, das die eingekoppelte Ultraschallenergie steuert und zwar entweder als Aus-/Einschaltung oder als Frequenzsteuerung, um dadurch eine möglichst schenlle und exakte Temperatureinstellung zu erhalten. Vorteilhafterweise dient hier die Ultraschalleinkoppelung zum Erwärmen sowie auch zum Rühren der Flüssigkeit, so daß zwei Funktionen mit nur einer Vorrichtung und einer Energiequelle realisiert werden. Da die Energiezufuhr in Form von Ultraschall trägheitslos steuerbar ist, entfällt das bei sonstigen Geräten übliche erforderliche Voreilen der Temperatur an der Erwärmungsstelle. Bei letzterem besteht nämlich die Gefahr eines Überschwingens. Die schräge Abstrahlfläche 11 hat zudem den Vorteil, daß im Behälter eine Zirkulation der Flüssigkeit erreicht wird, was außer der optimalen homogenen Durchmischung auch eine verbesserte Heizqualität mit sich bringt.

In Fig. 4 ist ein Ausführungsbeispiel des Gegenstandes der Erfindung zur Behandlung von Flüssigkeit dargestellt, die in geschlossenen Behältnissen, wie Kunststoffbeutel, enthalten sind. Derartige Flüssigkeiten können beispielsweise Infusionlösungen oder Blutkonserven sein, die von einer Kühltemperatur auf Körpertemperatur gebracht werden müssen und dabei keinerlei Schaden erleiden oder mit Luft in Berührung kommen dürfen.

Beispielhaft sind hier zwei Verbundschwinger, wie zu Fig. 1 beschrieben, auf nur einem Adapter 7 angeordnet, der wiederum an einem hier als Tank ausgebildeten Abstrahlglied 3 befestigt ist. Dieser Tank ist mit einem flüssigen Koppelmedium, beispielsweise Wasser, angefüllt. In diesen Tank 3 bzw. das Wasser 14 wird ein Kunststoffbeutel 15 gehängt, der mit der zu temperierenden Flüssigkeit gefüllt ist. Die Temperaturmessung erfolgt bei diesem Ausführungsbeispiel über ein Fenster 16 im Tank 3 mit einem Infrarottemperaturmeßgerät 17. Das Fenster 16 kann aus Germanium oder Silizium oder sonst einem infrarotdurchlässigen Medium bestehen. Der Beutel 15 mit dem zu behandelnden Medium wird über eine Halterung 18 von oben in den Tank 3 gehängt.

Das vorzugsweise als Kompensationsmeßgerät ausgebildete Infrarotmeßgerät 17 mißt die Flüssigkeitstemperatur im Beutel 15 und vergleicht sie mit einer Soll-Temperatur. Diese Infrarottemperaturmesser (Pyrometriegeräte), weisen einen Meßaufnehmer (Infrarotstrahlungsdetektor) auf, der auf Soll-Temperatur beispielsweise mit einem Pelletier-Thermostat gebracht wird und solange ein Signal abgibt, bis das zu messende Medium (Ist-Temperatur) die Soll-Temperatur erreicht hat. Da es sich bei der Anwendung meist um zellhaltige Flüssigkeiten handelt, wird mit einer hohen Frequenz gearbeitet, damit nicht durch Kavitation eine Zellschädigung entsteht. Da die Dichte von Wasser und Kunststoff nahezu gleich ist, ist ein verlustarmer Schwingungsdurchgang vom Adapter 7 über das Koppelmedium 14 (Wasser) zum zu temperierenden Medium im Beutel 15 gewährleistet.

Bekanntlich ist das Mischen von Flüssigkeiten innerhalb von verschlossenen Kunststoffbeuteln nahezu unmöglich, so daß sich gerade bei diesem Beispiel die Eigenart des Ultraschalls, Flüssigkeiten zu homogenisieren, entscheidend auswirkt.

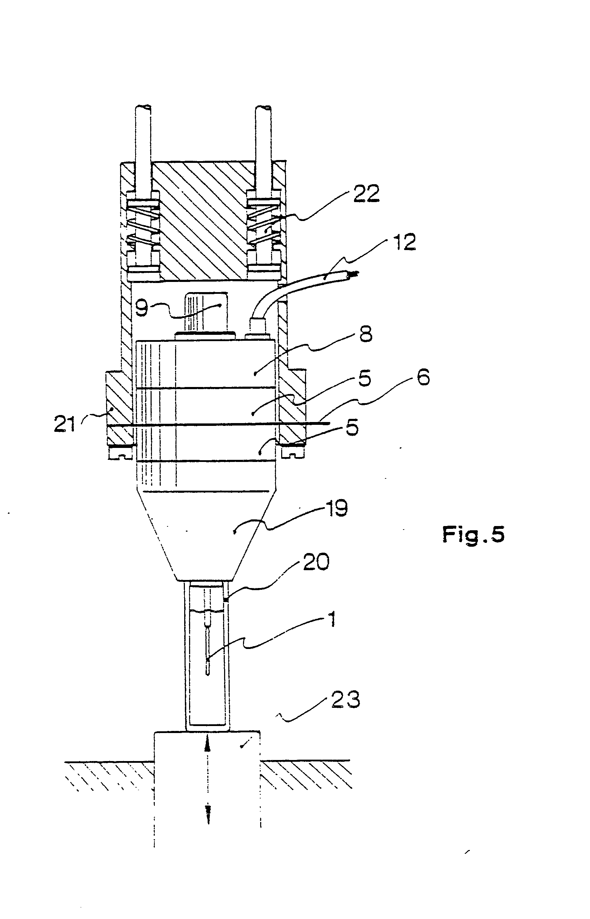

Bei dem in Fig. 5 dargestellten vierten Ausführungsbeispiel wird ein im Prinzip wie beim zweiten Ausführungsbeispiel (Fig. 3) aufgebauter Verbundschwinger mit achsgleichem Temperaturgeber verwendet, um durch Schwingungserregung von Behälterwandungen eine Temperierung zu erzielen. An den elektroakustischen Wandler 5 ist ein Ausstrahlglied oder Adapter 19 angeschlossen, dessen dem Wandler 5 abgewandte Seite der Form eines Behälters 20 angepaßt ist. Bei diesem Behälter kann es sich um eine oben offene Küvette handeln für eine photometrische Untersuchung im klinisch-chemischen oder biochemischen Bereich. Der Verbundschwinger ist hierfür in einer Halterung 21 beispielsweise über die Kontaktscheibe 6 befestigt, wobei die Halterung selbst an einem Schwingungswegausgleichselement 22 aufgehängt ist. Der Behälter 20 wird über eine Andrückvorrichtung 23 an das Abstrahlglied 19 kraftschlüssig angedrückt.

Durch die Schwingungen werden intensive Turbulenzen erzeugt, vor allem an den Wänden und in den Ecken des Behälters, an denen mit üblichen Rührverfahren keine Strömung erzeugt werden könnte. Aufgrund der auf den Behälter 20 übertragenen Ultraschallschwingung findet eine Homogenisierung und gleichmässige Erwärmung der Flüssigkeit statt, so daß im Grunde die Meßstelle irgendwo im Behälter angeordnet sein kann. Statt einem Berührungsmeßgeber 1 kann natürlich auch die Temperatur mit optischen Mitteln gemessen werden. In jedem Fall ist aufgrund der Ultraschallwirkung eine Temperaturmeßgenauigkeit von 0,01 °C oder besser möglich. Dies ist besonders bei reaktionkinetischen Untersuchungen von Bedeutung, bei denen der Temperaturfehler die erreichbare Analysengenauigkeit und die Taktzeiten von Automaten maßgeblich limitiert.

Erfindungsgemäß kann generell und nicht nur bei den dargestellten Beispielen die Temperaturwirkung durch eine Ein-/Ausschaltung des Ultraschalls oder aber durch eine Änderung der Schwingungsamplitude erzielt werden.

**Patentansprüche**

1. Verfahren zur Temperaturregelung einer Flüssigkeit mit elektrischen Mitteln und Steuerung der elektrischen Mittel unter Auswertung des Temperaturvergleichs der gemessenen Ist-Temperatur mit einer Soll-Temperatur und unter Änderung der an die Flüssigkeit abgegebenen Wärmeleistung, dadurch gekennzeichnet, daß die elektrischen Mittel zur Erzeugung akustischer Ultraschallwellen dienen und daß die Änderung der Ist-Temperatur entsprechend der vorgegebenen Solltemperatur durch Ändern der Schwingungsamplitude oder durch Ein- und Ausschalten des Ultraschalls erfolgt.

2. Vorrichtung zur Temperaturregelung einer ruhenden oder strömenden Flüssigkeit, mit einem topf- oder rohrförmigen Flüssigkeitsbehälter bestimmten Volumens, mit einem elektrischen Wärmeerzeuger für die im Behälter vorhandene Flüssigkeit, mit einem Temperaturgeber zur Messung der Ist-Temperatur und mit einem mit dem Temperaturgeber und dem Wärmeerzeuger elektrisch verbundenen elektronischen Steuergerät zur Änderung der Wärmeleistung nach Vergleich und Auswertung der gemessenen Ist-Temperatur mit einer vorgegebenen Soll-Temperatur, dadurch gekennzeichnet, daß der Behälter (2, 13, 15, 20) mit einem einen elektrischen Generator (5, 6, 7, 8, 9) zur Erzeugung der Resonanzfrequenz aufweisenden elektroakustischen Wandler (Ultraschallerzeuger) (5, 6, 7, 8, 9) verbunden ist, daß der Temperaturgeber (1) an einer beliebigen Stelle des Volumens angeordnet ist und daß durch das elektronische Steuergerät die Leistung des Generators bis zum Erreichen der Soll-Temperatur änderbar oder abschaltbar ist.

3. Vorrichtung nach Anspruch 2, dadurch gekennzeichnet, daß als Temperaturgeber (1) optische Mittel (17) (Infrarotstrahlungsgerät und dergl.) dienen, mit denen die Temperatur der Flüssigkeit berührungsfrei meßbar ist.

4. Vorrichtung nach Anspruch 2 oder 3, dadurch gekennzeichnet, daß zwischen Behälter (2, 15) und Ultraschallerzeuger (5) ein mit diesem unmittelbar verbundenes Ultraschallabstrahlglied (3, 19) vorgesehen ist, welches mit dem Behälter (2, 15) über ein Koppelmedium (4, 14) verbunden ist.

5. Vorrichtung nach Anspruch 4, dadurch gekennzeichnet, daß das Koppelmedium (14) Flüssigkeit, insbesondere Wasser ist.

6. Vorrichtung nach Anspruch 4, dadurch gekennzeichnet, daß das Koppelmedium (4) aus einem elastischen Wärmeisoliermaterial besteht.

7. Vorrichtung nach einem der Ansprüche 2 bis 6, dadurch gekennzeichnet, daß der Ultraschallerzeuger aus mindestens einem elektroakustischen Wandler (5) besteht, an den sich einerseits ein Abschlußglied (8) und andererseits ein zum beschallten Medium hin angeordneter Adapter (7, 10, 19) anschließt.

8. Vorrichtung nach einem der Ansprüche 2 bis 7, dadurch gekennzeichnet, daß die Flüssigkeit über den Behälter (2, 20) selbst der Ultraschallschwingung aussetzbar ist.

9. Vorrichtung nach Anspruch 8, dadurch gekennzeichnet, daß als Behälter ein Rohr (2) dient und daß die das Rohr durchströmende Flüssigkeit der Ultraschallschwingung aussetzbar ist, indem der Ultraschallerzeuger (5) radial zum Rohr (2) angeordnet ist und die Ultraschallwellen quer zur Rohrachse verlaufen.

10. Vorrichtung nach einem der Ansprüch 2 bis 7, dadurch gekennzeichnet, daß die Flüssigkeit in einem geschlossenen Behälter (15) untergebracht ist und daß der Behälter (15) in eine als Koppelmedium dienende Flüssigkeit (14) getaucht wird, die wiederum durch einen Tank (3) aufgenommen wird, dessen Tankwand als Abstrahlfläche eines Abstrahlgliedes dient.

## Claims

1. A method for controlling the temperature of a liquid by electric means, and for controlling the electric means by evaluating the comparison between the measured actual temperature and a predetermined desired temperature and by varying the thermal efficiency transmitted to the liquid, characterized in that the said electric means serve for generating acoustic ultrasonic waves and that the actual temperature is varied according to the predetermined desired temperature by varying the amplitude of oscillation, or by switching on and off the ultrasonic waves.

2. Apparatus for controlling the temperature of a stagnant or flowing liquid, comprising a pot or tube-shaped liquid container of a given volume, electric heat-generating means for heating the liquid present in the container, temperature-sensing means for measuring the actual temperature and an electronic control device which is electrically connected to the temperature sensor and the heat-generating means for varying the thermal efficiency after comparison and evaluation of the measured actual temperature and a predetermined desired temperature, characterized in that the said container (2, 13, 15, 20) is connected to an electroacoustic transducer (ultrasonic wave generator) (5, 6, 7, 8, 9) comprising an electric generator (5, 6, 7, 8, 9) for generating the resonance frequency, that the said temperature sensor (1) is arranged at any point of the volume and that the power of the generator can be varied until the predetermined temperature is reached, or switched off altogether, by the said electronic control device.

3. Apparatus according to claim 2, characterized in that optical means (17) (infrared lamp or the like) are provided as temperature sensor for measuring the temperature in a contactless manner.

4. Apparatus according to claim 2 or 3, characterized in that an ultrasonic wave radiation member (3, 19) is provided between the said container (2, 15) and the said ultrasonic wave generator (5) and connected to the latter directly and to the said container (2, 15) via a coupling medium (4, 14).

5. Apparatus according to claim 4, characterized in that the coupling medium (14) is a liquid, in particular water.

6. Apparatus according to claim 4, characterized in that the coupling medium (4) consists of an elastic thermally insulating material.

7. Apparatus according to any of claims 2 to 6, characterized in that the said ultrasonic wave generator comprises at least one electroacoustic transducer (5) which is followed on the one hand by a closure element (8) and, on the other hand, towards the liquid exposed to the ultrasonic wave, by an adapter (7, 10, 19).

8. Apparatus according to any of claims 2 to 7, characterized in that the liquid itself can be exposed to the ultrasonic waves via the container (2, 20).

9. Apparatus according to claim 8, characterized in that the said container is formed by a tube (2) and that the liquid flowing through the said tube can be exposed to the ultrasonic waves by arranging the ultrasonic wave generator (5) radially to the tube (2) so that the ultrasonic waves propagate transversely to the tube axis.

10. Apparatus according to any of claims 2 to 7, characterized in that the liquid is accommodated in a sealed container (15) and that the said container (15) is immersed in a liquid (14) serving as a coupling medium and being in turn contained in a tank (3) whose wall serves as radiation surface of a radiation member.

**Revendications**

1. Procédé pour le réglage de la température d'un liquide par des moyens électriques et commande des moyens électriques par évaluation de la comparaison de la température réelle mesurée avec une température de consigne, et par modification de la puissance calorifique délivrée au liquide, caractérisé en ce que les moyens électriques servent à la génération d'ondes acoustiques ultrasonores et que la modification de la température réelle est effectuée en fonction de la température de consigne prédéfinie, par variation de l'amplitude d'oscillation ou par mise en et hors service de l'ultrason.

2. Dispositif pour le réglage de la température d'un liquide au repos ou coulant avec un récipient en forme de pot ou de tube d'un volume défini, avec un générateur de chaleur pour le liquide disponible dans le récipient, avec un capteur de température pour la mesure de la température réelle et avec un appareil de commande électronique, relié électriquement au générateur de chaleur, pour la modification de la puissance calorifique après comparaison et évaluation de la température réelle mesurée avec une température de consigne prédéfinie, caractérisé en ce que le récipient (2, 13, 15, 20) est relié à un transducteur (générateur d'ultrasons) électro-acoustique (5, 6, 7, 8, 9) présentant un générateur électrique (5, 6, 7, 8, 9) pour la génération de la fréquence de résonance, en ce que le capteur de température (1) est disposé à un endroit quelconque du volume et en ce que, grâce à l'appareil de commande électronique, le rendement du générateur peut être varié ou interrompu jusqu'à obtention de la température de consigne.

3. Dispositif selon la revendication 2, caractérisé en ce que des moyens optiques (17) (appareil à rayonnement infrarouge et similaires), permettant de mesurer sans contact la température du liquide, sont utilisés comme capteur de température (1).

4. Dispositif selon la revendication 2 ou 3, caractérisé en ce que, entre le récipient (2, 15) et le générateur d'ultrasons (5), est prévu un élément de dégagement d'ultrasons (3, 19) relié au récipient (2, 15) par l'intermédiaire d'un moyen de couplage (4, 14).

5. Dispositif selon la revendication 4, caractérisé en ce que le moyen de couplage (14) est un liquide, essentiellement de l'eau.

6. Dispositif selon la revendication 4, caractérisé en ce que le moyen de couplage (4) consiste en un matériau élastique calorifuge.

7. Dispositif selon l'une des revendications 2 à 6, caractérisé en ce que le générateur d'ultrasons comprend au moins un transducteur (5) électro-acoustique, muni d'une part d'un élément terminal (8) et d'autre part d'un adaptateur (7, 10, 19) disposé en direction de l'agent exposé à l'action des ultrasons.

8. Dispositif selon l'une des revendications 2 à 7, caractérisé en ce que le liquide peut être exposé aux oscillations ultrasonores à travers le récipient (2, 20) même.

9. Dispositif selon la revendication 8, caractérisé en ce qu'un tube (2) sert de récipient et que le liquide coulant à travers le tube peut être exposé à l'oscillation ultrasonore, par le fait que le générateur d'ultrasons (5) est disposé radialement par rapport au tube et les ondes ultrasonores se déplacent transversalement à l'axe du tube.

10. Dispositif selon l'une des revendications 2 à 7, caractérisé en ce que le liquide est logé dans un récipient fermé (15) et que le récipient (15) est plongé dans un liquide (14) servant d'agent de couplage et qui est lui logé dans un réservoir (3) dont la paroi sert de surface radiante d'un élément de rayonnement.

Fig.2

Fig.1

Fig. 3

Fig. 4

0 149 628

Fig.5